**Europäisches Patentamt**

**European Patent Office**

**Office européen des brevets**

(11) Publication number: **0 423 330 A1**

# EUROPEAN PATENT APPLICATION
## published in accordance with Art. 158(3) EPC

(21) Application number: 89901590.3

(22) Date of filing: 19.01.89

(86) International application number:
PCT/JP89/00045

(87) International publication number:
WO 89/06524 (27.07.89 89/16)

(51) Int. Cl.⁵: **A61F 13/20**

(30) Priority: 22.01.88 JP 12330/88

(43) Date of publication of application:
24.04.91 Bulletin 91/17

(84) Designated Contracting States:
AT BE CH DE FR GB IT LI NL SE

(71) Applicant: SHIMATANI, Sumie
109-3, Koyato, Samukawa-machi
Kouza-gun, Kanagawa 253-01(JP)

(72) Inventor: SHIMATANI, Kazuo
109-3, Koyato Samukawa-machi
Kouza-gun Kanagawa 253-01(JP)

(74) Representative: Hillier, Peter et al
Reginald W. Barker & Co., 13, Charterhouse
Square
London, EC1M 6BA(GB)

(54) **TAMPON.**

(57) A tampon composed of a tampon body made of a stringy material is prepared by intertwining menstrual blood-absorbing threads, such as cotton threads of a high water absorptivity, and applying an external pressure to the formed tampon body so as to compress the same into a bullet. This tampon has a large surface area and a number of cavities among the intertwined and compressed threads. Accordingly the tampon can absorb the menstrual blood efficiently to swell. Thus the tampon can absorb the menstrual blood falling down along the wall of a vagina without fail, as it expands substantially to the volume which it had before the compression. In order to take out the tampon, a stringy member formed by intertwining reinforced threads is pulled out, so that the tampon can be removed from the vagina smoothly as it is loosened.

# TAMPON

## Background of the Invention

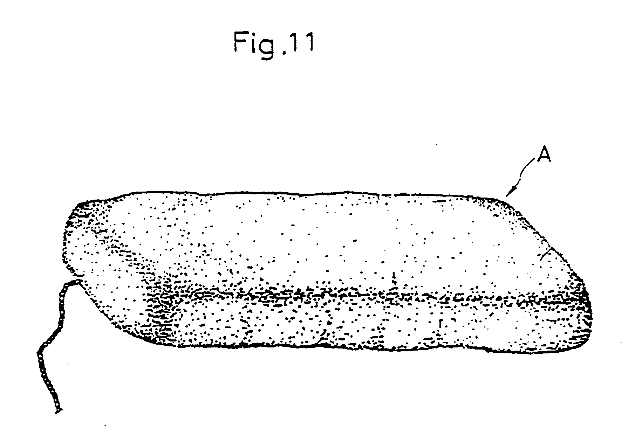

As shown in Fig. 11, a conventional tampon used for such as a sanitary tampon, comprises a tampon body A of wide width in the form of a belt by means of absorbing materials such as cotton wool, absorbing papers and the like. After the tampon body A is wound tightly in spiral so as not to get loose, it is formed into a bullet like shape by adding a pressure on the outer perihery of the body. In another way, the tampon body A is formed into a shape of a stick by compressing same in the upper and lower directions as well as in the lateral directions.

However, according to such a conventional tampon, since the tampon body A merely forms a continuous, simple and flat body, the tampon body A lacks a restoring force when it absorbs menstrual blood and expands inradial directions thereof. Therefore, it cannot wipe a stain sufficiently from the wall of the vagina.

## Summary of the invention

In view of the above-mentioned problems, the present invention has an object to provide a tampon which has a strong restoring force and surely absorbs the menstrual blood running on the wall of the vagina and also wipe a stain sufficiently from the wall of the vagina when the tampon is took out from the vagina.

To achieve the above-mentioned object, a tampon according to the present invention, comprises a tampon body with string like bodies which comprise intertwisted strings such as cotton yarns having absorbency of menstrual blood and which is compressed with external force in the longitudinal directions thereof and in the radial directions from the outer periphery thereof.

Therefore, according to the present invention, since the tampon body is formed by binding the string like bodies such as cotton wool having absorbency of menstrual blood and it is compressed to be a bullet like shape, when the tampon body absorbs the menstrual blood, it expands greatly due to a restoring force of each of the string like bodies to fit the wall of the vagina so as to be able to surely absorb the menstrual blood.

Further, since the tampon body has a great surface area by means of each of the string like bodies, it has a high ability of absorbency.

The present invention controls napping or piling of the tampon body to the utmost in comparison with the conventional tampon which is merely formed with cotton wool. Therefore, it is advantageous that it becomes easy to manufacture the tampon since it is no need to take a treatment for the outer surface of the tampon body.

Other advantages of this invention will be apparent from the following description with reference to the drawings.

## Brief Description of the Drawings

The drawings show embodiments of a tampon according to this invention in which:-
Fig. 1 is a perspective view of string like bodies forming the tampon,
Fig. 2 is a perspective view of the string like bodies in the state that it is bound in the form of a loop,
Fig. 3 is a perspective view of forming the bound string like bodies into a tampon body,
Fig. 4 is a perspective view of a tampon body as another type,
Fig. 5 and Fig. 6 are perspective views of the tampon bodies wound twistedly,
Fig. 7 is a perspective view of the tampon formed into a bullet like shape by compressing the twisted tamponbodies,
Fig. 8 is a perspective view of the tampon in the state that it is took out after use,
Fig. 9 is a perspective view of string like bodies forming a tampon according to another embodiment of the present invention,
Fig. 10 is a persepective view of the tampon bodies bound, and
Fig. 11 is a perspective view of a tampon body of a conventional type.

## Detailed Description of the Embodiments

The embodiments of the present invention will be described in detail with reference to the drawings.

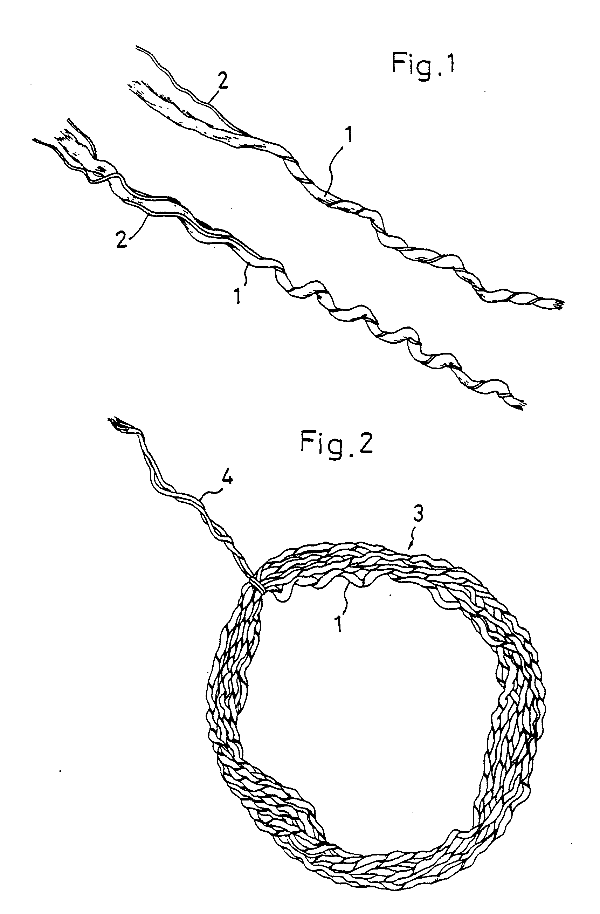

Fig. 1 shows string like bodies forming a tampon body 3 according to an embodiment of this invention.

The string like body 1 is formed by intertwisting strings such as cotton wool and the like, which has a great absorbency of menstrual blood, to have a diameter of, for instance, 2 mm or 3mm. Further, one or two strings 2 are entwisted with the outer periphery of the string like body 1 so as to increase a tensile strength in the longitudinal directions thereof.

The string forming the string like body 1 may be made of any material which just provide the above-mentioned function, and is not limitted in its material. Further, the reinforcing strings 2 are not limitted in tis material, too.

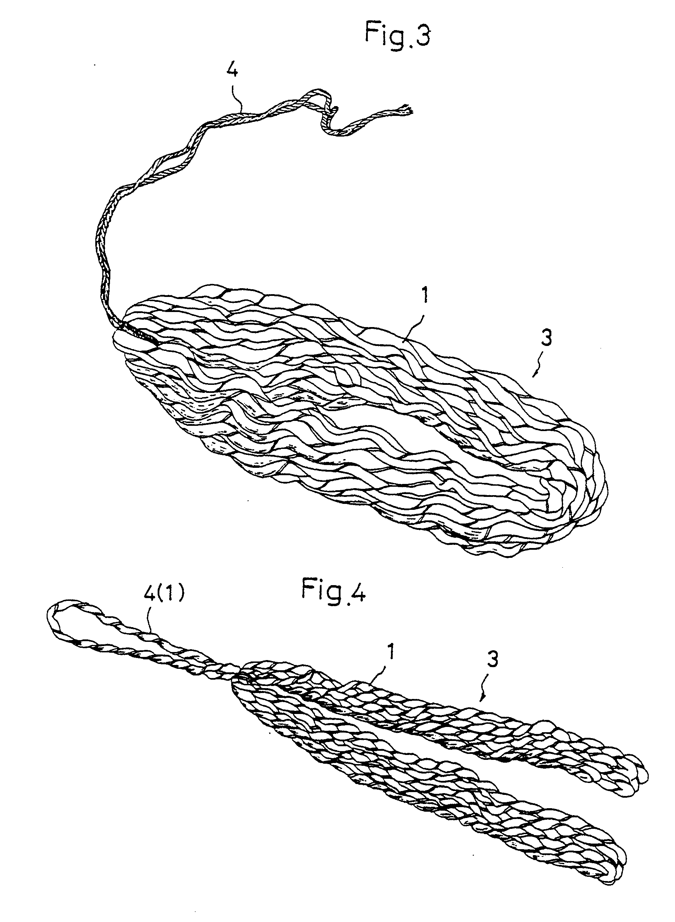

The string like bodies 1 are wound many times to be a loop to form a tampon body 3 having a suitable thickness, width and length (See Figs. 2 and 3). It is possible to just entwist thereinto the starting end portion and the closing end portion of the string like bodies 1 without tieing up both of the end portions.

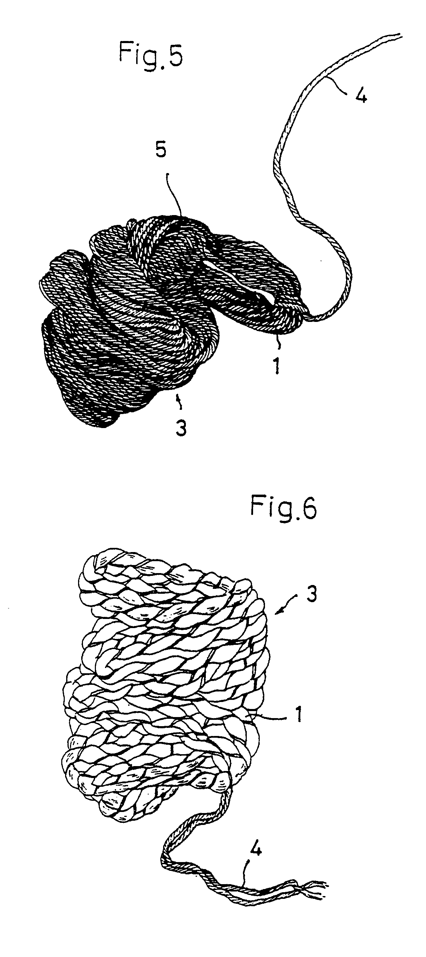

In the drawings, numeral 4 is a string for taking out the tampon. The string 4 may be served for the string like body 1 by projecting a portion of the string like body 1 from the tanpon body 3, as shown in Fig. 4. As shown in Figs. 5 and 6, the tampon body 3 is formed by intertwisting the string like bodies 1 in spiral so as to form a space slightly at a intermediate portion thereof and then in the state, compressed with external force added in the longitudinal directions and in the radial directions from the outer periphery thereof.

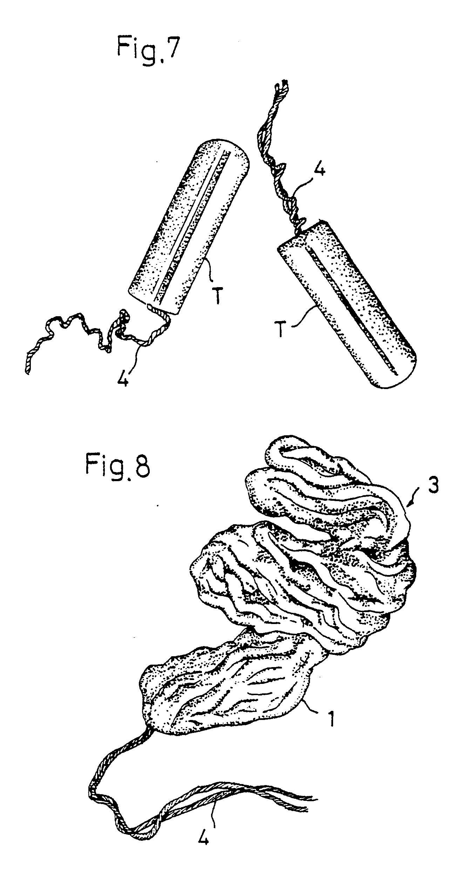

The compressed tampon T forms a bullet likeshape, as shown in Fig. 7.

In use, there is no difference comparing with the conventional tampon. When the tampon T absorbs the menstrual blood in the vagina, it gradually expands in the radial and longitudinal directions and returnsto its untwisted original shape of the tampon body 3. At the time, since the tampon body 3 is formed with a bunch of the string like bodies 1 which are superior in water-absorbency, it surely returns to its original shape rapidly by means of a restoring force of the string like bodies 1 when it absorbs the menstrual blood a little.

Further, as such a restoring function goes, the tampon body 3 increases its surface area greatly and therefore even a lot of menstrual blood may be absorbed smoothly.

Further, when the tampon body 3, which comprises a plurality of string like bodies 1, expands, their surfaces contact the wall of the vagina gently, and so it may also absorb the menstrual blood running on the wall of the vagina without leaking from the vagina.

After use, when the take-out string 4 is pulled, the tampon T is got loose from the state in Fig. 6 to the state in Fig. 8 and then smoothly took out from the vagina. In case that the take-out string 4 is made of a part of the string like body 1, the string like body 1 is not formed with only simple cotton yarns, but the reinforcing string 2 is entwisted with the outer periphery thereof, and therefore it has a sufficient tensile strength so that it may not be cut off at an intermediate portion thereof.

Of course, the tampon T formed with the string like bodies 1, may be compressed to be a bullet like shape by simply adding external force from the state in Fig. 3, as is used in the conventional tampon.

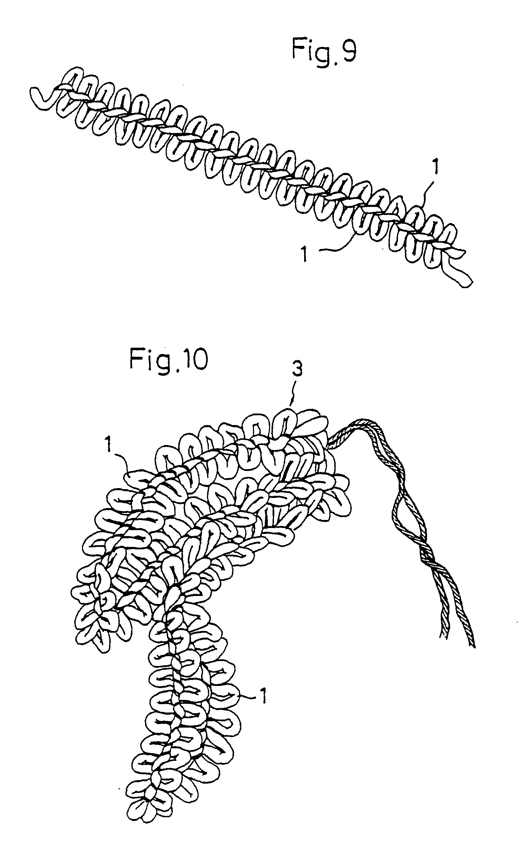

Further, when a plurality of the string like bodies are bound in a bunch, they may be bunched up as shown in Fig. 10 after they are weaved in advance in the state, for instance, as shown in Fig. 9, in order to make them expandable in the longitudinal or radial directions and also to be able to increase its surface area.

By utilizing the binding manner in this embodiment, it becomes possible to comprise a tampon body which provides a rapid and high restorebility.

## Claims

1. A tampon comprising a tampon body formed by string members intertwisting string like bodies consisting of cotton wool and the like having absorbency of menstrual blood wherein the tampon body is compressed with external force added in the longitudinal directions and in the radial directions from the outer periphery thereof.

2. The tampon as claimed in claim 1 wherein a reinforcing string or strings are entwisted with the outer periphery of the said string like bodies.

3. The tampon as claimed in claim 1 wherein the said tampon body is compressed with external force after giving a twist to the tampon body to form a spiral in the longitudinal directions thereof.

4. The tampon as claimed in claim 1 wherein at least one of the string like bodies is served for a take-out string to project from the end of the tampon body.

Fig.1

Fig.2

Fig.3

Fig.4

## Fig.5

## Fig.6

EP 0 423 330 A1

## Fig.7

## Fig.8

7

## Fig.9

## Fig.10

Fig.11

A

# INTERNATIONAL SEARCH REPORT

PCT/JP89/00045

**I. CLASSIFICATION OF SUBJECT MATTER** (if several classification symbols apply, indicate all) 6

According to International Patent Classification (IPC) or to both National Classification and IPC

Int. Cl$^4$     A61F13/20

**II. FIELDS SEARCHED**

| Minimum Documentation Searched 7 | |
|---|---|
| Classification System | Classification Symbols |
| IPC | A61F13/20 |

Documentation Searched other than Minimum Documentation
to the Extent that such Documents are Included in the Fields Searched 8

| | |
|---|---|
| Jitsuyo Shinan Koho | 1946 – 1988 |
| Kokai Jitsuyo Shinan Koho | 1971 – 1988 |

**III. DOCUMENTS CONSIDERED TO BE RELEVANT** 9

| Category * | Citation of Document, with indication, where appropriate, of the relevant passages 12 | Relevant to Claim No. 13 |
|---|---|---|
| A | JP, B1, 44-5520 (Kimberly-Clork Corp.) 7 March 1969 (07. 03. 69) (Family: none) | 1-4 |
| A | JP, U, 51-29794 (Anne Co., Ltd.) 3 March 1976 (03. 03. 76) (Family: none) | 1-4 |

* Special categories of cited documents: 10

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| April 5, 1989 (05. 04. 89) | April 17, 1989 (17. 04. 89) |
| International Searching Authority | Signature of Authorized Officer |
| Japanese Patent Office | |

Form PCT ISA 210 (second sheet) (January 1985)